Europäisches Patentamt

European Patent Office

Office européen des brevets

(19)

(11) Publication number: **0 299 528 B1**

# EUROPEAN PATENT SPECIFICATION

(12)

(45) Date of publication of patent specification: **25.11.92**

(51) Int. Cl.⁵: **A61K 31/71**, A61K 47/12, A61K 47/10, A61K 47/22, A61K 47/04

(21) Application number: **88111434.2**

(22) Date of filing: **15.07.88**

The file contains technical information submitted after the application was filed and not included in this specification

(54) **Doxorubicin hydrochloride nonaqueous solution.**

(30) Priority: **16.07.87 US 74189**

(43) Date of publication of application: **18.01.89 Bulletin 89/03**

(45) Publication of the grant of the patent: **25.11.92 Bulletin 92/48**

(84) Designated Contracting States: **AT BE CH DE ES FR GB GR IT LI LU NL SE**

(56) References cited:
EP-A- 0 010 437
EP-A- 0 219 784
GB-A- 2 178 311

(73) Proprietor: **Bristol-Myers Squibb Company 345 Park Avenue New York, N.Y. 10154(US)**

(72) Inventor: **Kaplan, Murray Arthur 1026 Glencove Road Syracuse, N.Y. 13206(US)**
Inventor: **Bogardus, Joseph Ballard, Dr. 8239 Penstock Way Syracuse, N.Y. 13104(US)**
Inventor: **Perrone, Robert Kevin 7353 Tomwood Drive Liverpool, N.Y. 13090(US)**

(74) Representative: **Kinzebach, Werner, Dr. Patentanwälte Reitstötter, Kinzebach und Partner Sternwartstrasse 4 Postfach 86 06 49 W-8000 München 86(DE)**

EP 0 299 528 B1

## Description

The present invention relates to stable, concentrated solutions of hydrochloride which may be diluted with sterile water or other suitable parenteral diluent for parenteral administration.

Doxorubicin is an antineoplastic antibiotic which is isolated from the fermentation of Streptomyces peucetius var. caesius. It can also be synthesized chemically from daunorubicin.

The commercial lyophilized dosage form of doxorubicin (marketed by Adria Laboratories as Adriamycin®) is supplied as the hydrochloride salt in 10, 20 and 50 mg vials in combination with lactose at 50, 100 and 250 mg, respectively. Vials are reconstituted with Sodium Chloride Injection, USP (0.9%) or Sterile Water for Injection, USP, to give a final concentration of 2 mg/ml of doxorubicin HCl.

It would be desirable to have a solution dosage form of doxorubicin HCl which would not require reconstitution prior to use. Improper reconstitution of a lyophilized product sometimes results in the formation of air-borne droplets ("blow-back") which, in the case of a potent antitumor agent such as doxorubicin HCl, may be a health hazard to the personnel making up the solution for injection. Also, production of the present lyophilized doxorubicin HCl dosage form is quite costly, and a solution dosage form would be expected to have a lower cost of goods.

Despite the advantages of a solution dosage form of doxorubicin HCl, this drug has not heretofore been available in such form because of its inherent instability in aqueous systems and its low solubility and/or instability in common non-aqueous systems. The manufacturer of Adriamycin® indicates that the reconstituted lyophilized solution is stable for only 24 hours at room temperature and 48 hours under refrigeration (4°C).

A study carried out by Hoffman, et al. and reported in Am. J. Hosp. Pharm. 36: 1536-1538, 1979 indicates that reconstituted solutions of doxorubicin HCl are stable (≤10% potency loss) for up to six months when refrigerated at 4°C. Ketchum, et al. in Am. J. Intrav. Ther. Clin. Nutri. 8: 15-18, 1981, state that a reconstituted solution of doxorubicin HCl in 0.9% NaCl solution remains stable at room temperature or 5°C for seven days. Despite such indications that reconstituted doxorubicin HCl may be sufficiently stable for up to six months at 4°C, this is still undesirable for a commercial solution dosage form.

Janssen, et al. in Int. J. Pharmaceutics 23: 1-11, 1985, report that doxorubicin HCl appears to have its maximum stability at pH 4(Tris or phosphate buffers using HCl to adjust pH). Poochikian, et al. in Am. J. Hosp. Pharm. 38: 483-486, 1981, note that the stability of doxorubicin HCl is pH dependent and that the optimum stability is achieved when the reconstitution solvent is 5% Dextrose Injection, USP, having a pH∿4.5.

U.K. Patent Application 2,178,311A discloses solutions of doxorubicin HCl in nonaqueous solvents or mixtures of such solvents with water. Among the solvents mentioned is propylene glycol, although this is not indicated as being one of the preferred solvents. There is no disclosure, however, of use of the antioxidants, sodium formaldehyde bisulfite, tert-butylhydroquinone and α-tocopherol which we have found imparts unexpectedly improved stability at elevated temperatures.

It is an object of the present invention to provide a doxorubicin hydrochloride solution dosage form which is stable (≤10% potency loss) for at least 18 months at room temperature and at least four months at 37°C, which contains at least 5 mg of doxorubicin HCl per ml of solution and which can be diluted with water or other aqueous vehicle for parenteral administration.

## SUMMARY OF THE INVENTION

The present invention provides a stable, sterile doxorubicin hydrochloride solution in a sealed container suitable for dilution with sterile water or other aqueous vehicle for parenteral administration, said solution containing doxorubicin hydrochloride, 0.1 to 2 mg/ml of an antioxidant selected from α-tocopherol, sodium formaldehyde bisulfite and tert-butylhydroquinone and an amount of citric acid necessary to provide a solution pH in the range of 3-5 in a solvent medium consisting of 90-100% (v/v) of propylene glycol, 1,2-dihydroxybutane or 1,3-dihydroxypropane and 0-10% (v/v) water.

This invention relates to stable, concentrated, solutions of doxorubicin hydrochloride suitable, upon dilution with sterile water other suitable aqueous vehicle, for parenteral administration. Depending on the solvent medium employed, concentrations of from 5 to 20 mg/ml doxorubicin hydrochloride are used in the solutions. More particularly, concentrations of from 5 to 10 mg/ml are utilized with neat propylene glycol, 1,2-dihydroxybutane or 1,3-dihydroxypropane, and concentrations of from 5 to 20 mg/ml are used when 5-10% (v/v) water is mixed with the above-mentioned propylene glycol, 1,2-dihydroxybutane or 1,3-dihydroxypropane solvents.

Doxorubicin HCl at room temperature has a solubility of less than 5 mg/ml in propylene glycol, 1,2-dihydroxybutane and 1,3-dihydroxypropane. Upon short-term (0.5-1 hour) warming (60-80°C) with vigorous stirring, however, solubility of up to about 10 mg/ml can be achieved with no potency loss. The doxorubicin HCl remains in solution when stored at 4°C to -20°C and seeding of the solutions with crystalline doxorubicin HCl does not affect crystallization. Solubility of doxorubicin HCl can be increased to 20 mg/ml in the above-mentioned solvents by addition of 5-10% (v/v) water.

Solubilities of doxorubicin HCl in other solvents such as polyethylene glycols, ethanol, benzyl alcohol, dimethylisosorbide, ethyl lactate, Solketol®, Cremophors®, and Pluronics® were less than 5 mg/ml with heating and thus these solvents did not meet our minimum 5 mg/ml criterion for a solution form of doxorubicin HCl. Other solvents tested such as glycerol formal, 2-pyrrolidinone and 1-methyl-2-pyrrolidinone produced solutions of 5 mg/ml or greater but were chemically unstable.

One critical component of the solutions is pH with a pH range of about 3-5 being necessary for acceptable long-term stability. The pH is measured on solutions diluted to 1 mg/ml with water or 70% water/30% acetonitrile (v/v). While a number of acids may be used to achieve this pH range, we have found that optimum stability is achieved with citric acid since this acid acts as a metal chelation agent as well as provided pH control. An amount of citric acid is used which will provide a solution pH in the range of 3-5, preferably 3-4.5. Generally a concentration of citric acid of 0.2 mg/ml will provide this pH control.

It is also necessary for optimum stability of the solutions, especially at elevated temperatures which may be encountered at some time during the life of the product, e.g. during shipping, to utilize a particular antioxidant. We have tested a number of antioxidants including $\alpha$-tocopherol, tert-butylhydroquinone (TBHQ), sodium formaldehyde bisulfite, butylated hydroxy anisole (BHA), butylated hydroxy toluene (BHT), sodium bisulfite and propyl gallate. Only $\alpha$-tocopherol, tert-butyl-hydroquinone and sodium formaldehyde bisulfite resulted in the desired stability at elevated temperatures as well as at room temperature (25°C) or below. Generally an amount of $\alpha$-tocopherol, tert-butylhydroquinone or sodium formaldehyde bisulfite is employed in the range of 0.1-2 mg/ml. The preferred concentration of antioxidant is 0.5-2 mg/ml with 2 mg/ml being most preferred.

In our studies the use of buffers such as acetate and citrate buffers had a deleterious effect on stability of the solutions and the presence of surfactants such as Tween®-80 did not improve stability.

Despite reports of potency losses of doxorubicin HCl solutions via photolytic degradation and adsorption to container walls, we have not noted any significant loss of potency due to container adsorption or sensitivity to light with our solutions. Glass vials may conveniently be used as the container.

We have also found that deaeration of the solvent medium, e.g. by heating to 100°C and/or vacuum prior to formulation and nitrogen purging and deaeration of the filled final formulation vials before they are sealed contributes to optimum stability and is preferred. Nitrogen purging is a particularly preferred aspect of our solutions.

As an example of a stable non-aqueous solution of doxorubicin HCl, 20 mg/ml doxorubicin hydrochloride, 0.2 mg/ml citric acid (anhydrous), 2.0 mg/ml of TBHQ and a solvent medium consisting of deaerated propylene glycol/water (90:10 v/v) are added to a Type I flint vial, the vial is purged with nitrogen and then sealed with a suitable teflon-coated stopper. Another preferred embodiment consists of a solution wherein 5 mg/ml doxorubicin HCl, 0.2 mg/ml citric acid, 1 mg/ml of TBHQ and neat deaerated propylene glycol are added to a Type I flint vial. The vial is purged with nitrogen and then sealed with a suitable teflon-coated stopper. Alternatively, sodium formaldehyde bisulfite or $\alpha$-tocopherol may be used as the antioxidant and/or the solvent medium may be deaerated 1,2-dihydroxybutane or 1,3-dihydroxypropane or a deaerated mixture of 1,2-dihydroxybutane/water or 1,3-dihydroxypropane/water.

Elevated temperature studies indicate that our doxorubicin HCl non-aqueous solutions have an acceptable shelf-life (≦10% potency loss) of at least 18 months at room temperature and at least four months at 37°C.

The solutions provided by the present invention may be diluted with sterile water or other conventional sterile aqueous vehicle (e.g. saline solution) to a concentration of 2 mg/ml or less and are then employed for the treatment of cancer in the same manner as the present reconstituted lyophilized dosage form.

## Claims

## Claims for the following Contracting States : AT, BE, CH, DE, FR, GB, IT, LI, LU, NL, SE

1. A pharmaceutical formulation of a stable, sterile, solution of doxorubicin hydrochloride in a sealed container, said solution containing from 5 to 10 mg/ml of doxorubicin hydrochloride, 0.1 to 2 mg/ml of an antioxidant selected from $\alpha$-tocopherol, sodium formaldehyde bisulfite and tert-butylhydroquinone and an amount of citric acid necessary to provide a solution pH in the range of 3 to 5 in a solvent medium consisting of 90-100% (v/v) propylene glycol, 1,2-dihydroxybutane or 1,3-dihydroxypropane and 0-10% (v/v) water.

2. A formulation according to Claim 1 wherein a deaerated solvent medium is employed and the solution is subjected to nitrogen purging before the container is sealed.

3. A formulation according to Claim 1 or 2 wherein the solvent medium is neat propylene glycol.

4. A formulation according to Claim 1 or 2 wherein the solvent medium is neat 1,3-dihydroxypropane.

5. A formulation according to Claim 1 or 2 wherein the solvent medium is neat 1,2-dihydroxybutane.

6. A formulation according to Claim 1 or 2 wherein the solvent medium is propylene glycol/water (90:10 v/v).

7. A formulation according to Claim 1 or 2 wherein the solvent medium is propylene glycol/water (95:5 v/v).

8. A formulation according to Claim 1 or 2 wherein the solvent medium is 1,2-dihydroxybutane/water (90-95:5-10 v/v).

9. A formulation according to Claim 1 or 2 wherein the solvent medium is 1,3-dihydroxypropane/water (90-95:5-10 v/v).

10. A pharmaceutical formulation of a stable, sterile, solution of doxorubicin hydrochloride in a sealed container, said solution containing from 5 to 20 mg/ml of doxorubicin hydrochloride, 0.1 to 2 mg/ml of an antioxidant selected from $\alpha$-tocopherol, sodium formaldehyde bisulfite and tert-butylhydroquinone and an amount of citric acid necessary to provide a solution pH in the range of 3 to 5 in a solvent medium consisting of about 90-95% (v/v) propylene glycol, 1,2-dihydroxybutane or 1,3-dihydroxypropane and 5-10% (v/v) water.

11. A formulation according to Claim 10 wherein a deaerated solvent medium is employed and the solution is subjected to nitrogen purging before the container is sealed.

12. A formulation according to Claim 10 or 11 wherein the solvent medium is propylene glycol/water (90:10 v/v).

13. A formulation according to Claim 10 or 11 wherein the solvent medium is propylene glycol/water (95:5 v/v).

14. A formulation according to Claim 10 or 11 wherein the solvent medium is 1,2-dihydroxybutane/water (90-95:5-10 v/v).

15. A formulation according to Claim 10 or 11 wherein the solvent medium is 1,3-dihydroxypropane/water (90-95:5-10 v/v).

**Claims for the following Contracting States : ES, GR**

1. A process for preparing a pharmaceutical formulation of a stable, sterile solution of doxorubicin hydrochloride in a sealed container, which comprises dissolving doxorubicin hydrochloride, an antioxidant selected from $\alpha$-tocopherol, sodium formaldehyde bisulfite and tert-butylhydroquinone, and citric acid in a solvent medium consisting of 90-100% (v/v) propylene glycol, 1,2-dihydroxybutane or 1,3-dihydroxypropane and 0-10% (v/v) water in an amount that the solution contains 5 to 10 mg/ml of doxorubicin hydrochloride, 0.1 to 2 mg/ml of said antioxidant, and has a pH in the range of 3 to 5.

2. A process according to Claim 1 wherein a deaerated solvent medium is employed and the solution is subjected to nitrogen purging before the container is sealed.

3. A process according to Claim 1 or 2 wherein the solvent medium is neat propylene glycol.

4. A process according to Claim 1 or 2 wherein the solvent medium is neat 1,3-dihydroxypropane.

5. A process according to Claim 1 or 2 wherein the solvent medium is neat 1,2-dihydroxybutane.

6. A process according to Claim 1 or 2 wherein the solvent medium is propylene glycol/water (90:10 v/v).

7. A process according to Claim 1 or 2 wherein the solvent medium is propylene glycol/water (95:5 v/v).

8. A process according to Claim 1 or 2 wherein the solvent medium is 1,2-dihydroxybutane/water (90-95:5-10 v/v).

9. A process according to Claim 1 or 2 wherein the solvent medium is 1,3-dihydroxypropane/water (90-95:5-10 v/v).

10. A process for preparing a pharmaceutical formulation of a stable, sterile solution of doxorubicin hydrochloride in a sealed container, which comprises dissolving doxorubicin hydrochloride, an antioxidant selected from $\alpha$-tocopherol, sodium formaldehyde bisulfite and tert-butylhydroquinone, and citric acid in a solvent medium consisting of 90-95% (v/v) propylene glycol, 1,2-dihydroxybutane or 1,3-dihydroxypropane and 5-10% (v/v) water in an amount that the solution contains 5 to 10 mg/ml of doxorubicin hydrochloride, 0.1 to 2 mg/ml of said antioxidant, and has a pH in the range of 3 to 5.

11. A process according to Claim 10 wherein a deaerated solvent medium is employed and the solution is subjected to nitrogen purging before the container is sealed.

12. A process according to Claim 10 or 11 wherein the solvent medium is propylene glycol/water (90:10 v/v).

13. A process according to Claim 10 or 11 wherein the solvent medium is propylene glycol/water (95:5 v/v).

14. A process according to Claim 10 or 11 wherein the solvent medium is 1,2-dihydroxybutane/water (90-95:5-10 v/v).

15. A process according to Claim 10 or 11 wherein the solvent medium is 1,3-dihydroxypropane/water (90-95:5-10 v/v).

**Patentansprüche**
**Patentansprüche für folgende Vertragsstaaten : AT, BE, CH, DE, FR, GB, IT, LI, LU, NL, SE**

1. Pharmazeutische Formulierung aus einer stabilen, sterilen Doxorubicinhydrochlorid-Lösung in einem geschlossenen Behälter, wobei die Lösung 5 bis 10 mg/ml Doxorubicinhydrochlorid, 0,1 bis 2 mg/ml eines Antioxidationsmittels, ausgewählt unter $\alpha$-Tocopherol, Natriumformaldehydbisulfit und tert-Butylhydrochinon, und die zur Einstellung des pH-Wertes der Lösung im Bereich von 3 bis 5 erforderliche Menge Zitronensäure in einem Lösungsmittelmedium, bestehend aus 90-100% (V/V) Propylenglycol, 1,2-Dihydroxybutan oder 1,3-Dihydroxypropan und 0-10% (V/V) Wasser, enthält.

2. Formulierung nach Anspruch 1, worin ein entgastes Lösungsmittelmedium verwendet wird und die Lösung mit Stickstoff gespült wird, bevor der Behälter verschlossen wird.

3. Formulierung nach Anspruch 1 oder 2, worin das Lösungsmittelmedium reines Propylenglycol ist.

4. Formulierung nach Anspruch 1 oder 2, worin das Lösungsmittelmedium reines 1,3-Dihydroxypropan ist.

5. Formulierung nach Anspruch 1 oder 2, worin das Lösungsmittelmedium reines 1,2-Dihydroxybutan ist.

6. Formulierung nach Anspruch 1 oder 2, worin das Lösungsmittelmedium Propylenglycol/Wasser (90:10 V/V) ist.

7. Formulierung nach Anspruch 1 oder 2, worin das Lösungsmittelmedium Propylenglycol/Wasser (95:5 V/V) ist.

8. Formulierung nach Anspruch 1 oder 2, worin das Lösungsmittelmedium 1,2-Dihydroxybutan/Wasser (90-95:5-10 V/V) ist.

9. Formulierung nach Anspruch 1 oder 2, woran das Lösungsmittelmedium 1,3-Dihydroxypropan/Wasser (90-95:5-10 V/V) ist.

10. Pharmazeutische Formulierung aus einer stabilen, sterilen Doxorubicinhydrochlorid-Lösung in einem geschlossenen Behälter, wobei die Lösung 5 bis 20 mg/ml Doxorubicinydrochlorid, 0,1 bis 2 mg/ml eines Antioxidationsmittels, ausgewählt unter $\alpha$-Tocopherol, Natriumformaldehydbisulfit und tert-Butylhydrochinon, und die zur Einstellung des pH-Wertes der Lösung im Bereich von 3 bis 5 erforderliche Menge Zitronensäure in einem Lösungsmittelmedium, bestehend aus 90-95% (V/V) Propylenglycol, 1,2-Dihydroxybutan oder 1,3-Dihydroxypropan und 5-10% (V/V) Wasser, enthält.

11. Formulierung nach Anspruch 10, worin ein entgastes Lösungsmittelmedium verwendet wird und die Lösung mit Stickstoff gespült wird, bevor der Behälter verschlossen wird.

12. Formulierung nach Anspruch 10 oder 11, worin das Lösungsmittelmedium Propylenglycol/Wasser (90:10 V/V) ist.

13. Formulierung nach Anspruch 10 oder 11, worin das Lösungsmittelmedium Propylenglycol/Wasser (95:5 V/V) ist.

14. Formulierung nach Anspruch 10 oder 11, worin das Lösungsmittelmedium 1,2-Dihydroxybutan/Wasser (90-95:5-10 V/V) ist.

**15.** Formulierung nach Anspruch 10 oder 11, worin das Lösungsmittelmedium 1,3-Dihydroxypropan/Wasser (90-95:5-10 V/V) ist.

**Patentansprüche für folgende Vertragsstaaten : ES, GR**

**1.** Verfahren zur Herstellung einer pharmazeutischen Formulierung aus einer stabilen, sterilen Doxorubicinhydrochlorid-Lösung in einem geschlossenen Behälter, wobei man Doxorubicinhydrochlorid, ein Antioxidationsmittel, ausgewählt unter α-Tocopherol, Natriumformaldehydbisulfit und tert-Butylhydrochinon, und Zitronensäure in einem Lösungsmittelmedium, bestehend aus 90-100% (V/V) Propylenglycol, 1,2-Dihydroxybutan oder 1,3-Dihydroxypropan und 0-10% (V/V) Wasser in einer Menge löst, daß die Lösung 5-10 mg/ml Doxorubicinhydrochlorid, 0,1 bis 2 mg/ml des Antioxidationsmittels enthält und einen pH im Bereich von 3 bis 5 hat.

**2.** Verfahren nach Anspruch 1, worin ein entgastes Lösungsmittelmedium verwendet wird und die Lösung mit Stickstoff gespült wird, bevor der Behälter verschlossen wird.

**3.** Verfahren nach Anspruch 1 oder 2, worin das Lösungsmittelmedium reines Propylenglycol ist.

**4.** Verfahren nach Anspruch 1 oder 2, worin das Lösungsmittelmedium reines 1,3-Dihydroxypropan ist.

**5.** Verfahren nach Anspruch 1 oder 2, worin das Lösungsmittelmedium reines 1,2-Dihydroxybutan ist.

**6.** Verfahren nach Anspruch 1 oder 2, worin das Lösungsmittelmedium Propylenglycol/Wasser (90:10 V/V) ist.

**7.** Verfahren nach Anspruch 1 oder 2, worin das Lösungsmittelmedium Propylenglycol/Wasser (95:5 V/V) ist.

**8.** Verfahren nach Anspruch 1 oder 2, worin das Lösungsmittelmedium 1,2-Dihydroxybutan/Wasser (90-95:5-10 V/V) ist.

**9.** Verfahren nach Anspruch 1 oder 2, worin das Lösungsmittelmedium 1,3-Dihydroxypropan/Wasser (90-95:5-10 V/V) ist.

**10.** Verfahren zur Herstellung einer pharmazeutischen Formulierung aus einer stabilen, sterilen Doxorubicinhydrochlorid-Lösung in einem geschlossenen Behälter, wobei man Doxorubicinhydrochlorid, ein Antioxidationsmittel, ausgewählt unter α-Tocopherol, Natriumformaldehydbisulfit und tert-Butylhydrochinon, und Zitronensäure in einem Lösungsmittelmedium, bestehend aus 90-95% (V/V) Propylenglycol, 1,2-Dihydroxybutan oder 1,3-Dihydroxypropan und 5-10% Wasser (V/V) in einer Menge löst, daß die Lösung 5-10 mg/ml Doxorubicinhydrochlorid, 0,1 bis 2 mg/ml des Antioxidationsmittels enthält und einen pH im Bereich von 3 bis 5 hat.

**11.** Verfahren nach Anspruch 10, worin ein entgastes Lösungsmittelmedium verwendet wird und die Lösung mit Stickstoff gespült wird, bevor der Behälter verschlossen wird.

**12.** Verfahren nach Anspruch 10 oder 11, worin das Lösungsmittelmedium Propylenglycol/Wasser (90:10 V/V) ist.

**13.** Verfahren nach Anspruch 10 oder 11, worin das Lösungsmittelmedium Propylenglycol/Wasser (95:5 V/V) ist.

**14.** Verfahren nach Anspruch 10 oder 11, worin das Lösungsmittelmedium 1,2-Dihydroxybutan/Wasser (90-95:5-10 V/V) ist.

**15.** Verfahren nach Anspruch 10 oder 11, worin das Lösungsmittelmedium 1,3-Dihydroxypropan/Wasser (90-95:5-10 V/V) ist.

**Revendications**
**Revendications pour les Etats contractants suivants : AT, BE, CH, DE, FR, GB, IT, LI, LU, NL, SE**

**1.** Formule pharmaceutique d'une solution stable et stérile de chlorhydrate de doxorubicine dans un conteneur scellé, ladite solution contenant 5 à 10 mg/ml de chlorhydrate de doxorubicine, 0,1 à 2 mg/ml d'un antioxydant choisi parmi α-tocophérol, formaldéhyde bisulfite de sodium et tert-butylhydroquinone et une quantité d'acide citrique nécessaire pour donner un pH de la solution compris entre 3 et 5, dans un milieu solvant consistant en 90-100% (volume/volume) de propylène glycol, de 1,2-dihydroxybutane ou de 1,3-dihydroxypropane et 0-10% (volume/volume) d'eau.

**2.** Formule selon la revendication 1, où un milieu solvant désaéré est employé et la solution est soumise à une purge à l'azote avant que le conteneur ne soit scellé.

**3.** Formule selon la revendication 1 ou 2, où la milieu solvant est du propylène glycol à l'état pur.

**4.** Formule selon la revendication 1 ou 2, où le milieu solvant est le 1,3-dihydroxypropane à l'état pur.

**5.** Formule selon la revendication 1 ou 2, où le milieu solvant est le 1,2-dihydroxybutane à l'état pur.

**6.** Formule selon la revendication 1 ou 2, où le milieu solvant est du propylène glycol/eau (90:10, volume/volume).

**7.** Formule selon la revendication 1 ou 2, où le milieu solvant est du propylène glycol/eau (95:5, volume/volume).

**8.** Formule selon la revendication 1 ou 2, où le milieu solvant est du 1,2-dihydroxybutane/eau (90-95:5-10 volume/volume).

**9.** Formule selon la revendication 1 ou 2, où le milieu solvant est du 1,3-dihydroxypropane/eau (90-95: 5-10 volume/volume).

**10.** Formule pharmaceutique d'une solution stable et stérile de chlorhydrate de doxorubicine dans un conteneur scellé, ladite solution contenant 5 à 20 mg/ml de chlorhydrate de doxorubicine, de 0,1 à 2 mg/ml d'un antioxydant choisi parmi α-tocophérol, formaldéhyde bisulfite de sodium et tert-butylhydroquinone et une quantité d'acide citrique nécessaire pour donner un pH de la solution compris entre 3 et 5 dans un milieu solvant consistant en environ 90-95% (volume/volume) de propylène glycol, 1,2-dihydroxybutane ou 1,3-dihydroxypropane et 5-10% (volume/volume) d'eau.

**11.** Formule selon la revendication 10, où on emploie un milieu solvant désaéré et la solution est soumise à une purge à l'azote avant que le conteneur ne soit scellé.

**12.** Formule selon la revendication 10 ou 11, où le milieu solvant est le propylène glycol/eau (90:10, volume/volume).

**13.** Formule selon la revendication 10 ou 11, où le milieu solvant est du propylène glycol/eau (95:5, volume/volume).

**14.** Formule selon la revendication 10 ou 11 où le milieu solvant est le 1,2-dihydroxybutane/eau (90-95:5-10 volume/volume).

**15.** Formule selon la revendication 10 ou 11, où le milieu solvant est le 1,3-dihydroxypropane/eau (90-95:5-10 volume/volume).

**Revendications pour les Etats contractants suivants : ES, GR**

**1.** Procédé de préparation d'une formule pharmaceutique d'une solution stable et stérile de chlorhydrate de doxorubicine dans un conteneur scellé, qui consiste à dissoudre le chlorhydrate de doxorubicine, un antioxydant choisi parmi l'α-tocophérol, le formaldéhyde bisulfite de sodium et la tert-butylhydroquinone et de l'acide citrique dans un milieu solvant consistant en 90-100% (volume/volume) de propylène glycol, de 1,2-dihydroxybutane ou de 1,3-dihydroxypropane et 0-10% (volume/volume) d'eau en une quantité telle que la solution contiennent 5 à 10 mg/ml de chlorhydrate de doxorubicine, 0,1 à 2 mg/ml dudit antioxydant et ait un pH compris entre 3 et 5.

**2.** Procédé selon la revendication 1, où on emploie un milieu solvant désaéré et la solution est soumise à une purge à l'azote avant que le conteneur ne soit scellé.

**3.** Procédé selon la revendication 1 ou 2, où le milieu solvant est du propylène glycol à l'état pur.

**4.** Procédé selon la revendication 1 ou 2, où le milieu solvant est du 1,3-dihydroxypropane à l'état pur.

**5.** Procédé selon la revendication 1 ou 2, où le milieu solvant est du 1,2-hydroxybutane à l'état pur.

**6.** Procédé selon la revendication 1 ou 2, où le milieu solvant est du propylène glycol/eau (90:10, volume/volume).

**7.** Procédé selon la revendication 1 ou 2, où le milieu solvant est du propylène glycol/eau (95:5, volume/volume).

**8.** Procédé selon la revendication 1 ou 2, où le milieu solvant est du 1,2-dihydroxybutane/eau (90-95:5-10, volume/volume).

**9.** Procédé selon la revendication 1 ou 2, où le milieu solvant est du 1,3-dihydroxypropane/eau (90-95:5-10, volume/volume).

**10.** Procédé de préparation d'une formule pharmaceutique d'une solution stable et stérile de chlorhydrate de doxorubicine dans un conteneur scellé, qui consiste à dissoudre le chlorhydrate de doxorubicine, un antioxydant choisi parmi α-tocophérol, formaldéhyde bisulfite de sodium et tert-butylhydroquinone et de l'acide citrique dans un milieu solvant consistant en 90-95% (volume/volume) de propylène glycol, de 1,2-dihydroxybutane ou de 1,3-dihydroxypropane et 5-10% (volume/volume) d'eau en une quantité telle que la solution contienne 5 à 10 mg/ml de chlorhydrate de doxorubicine, 0,1 à 2 mg/ml dudit antioxydant et ait un pH compris entre 3 et 5.

11. Procédé selon la revendication 10, où on emploie un milieu solvant désaéré et la solution est soumise à une purge à l'azote avant que le conteneur ne soit scellé.

12. Procédé selon la revendication 10 ou 11, où le milieu solvant est le propylène glycol/ eau (90:10, volume/volume).

13. Procédé selon la revendication 10 ou 11, où le milieu solvant est du propylène glycol/eau (95:5, volume/volume).

14. Procédé selon la revendication 11, où le milieu solvant est le 1,2-dihydroxybutane/eau (90-95:5-10, volume/volume).

15. Procédé selon la revendication 10 ou 11, où le milieu solvant est le 1,3-dihydroxypropane/eau (90-95:5-10, volume/volume).